# EUROPEAN PATENT SPECIFICATION

(11) **EP 0 900 551 B1**
(45) Date of publication and mention of the grant of the patent: **11.05.2005**
(21) Application number: 98830261.8
(22) Date of filing: 29.04.1998
(51) Int. Cl.: A61F 2/06

(54) **The structure of a radially expandable stent**
STRUKTUR EINES RADIAL EXPANDIERBAREN STENTS
Structure d'un stent expansible radialement

(30) Priority: 03.09.1997 IT BS970074
(43) Date of publication of application: 10.03.1999
(73) Proprietor: FOGAZZI DI VENTURELLI ANDREA & C. S.N.C., 25062 Concesio (Brescia) (IT)
(72) Inventor: Venturelli, Andrea, 25062 Concesio, Brescia (IT); Venturelli, Luigi, 25062 Concesio, Brescia (IT)
(74) Representative: Crippa, Paolo Ernesto

(56) References cited:
- WO-A-98/07385
- US-A- 4 856 516
- US-A- 4 994 071
- US-A- 5 135 536
- US-A- 5 217 483
- US-A- 5 562 697

## Description

The present invention refers to a metal stent that can be implanted in all the tubular ways of the human body or animals, for all applications, in particular to improve or maintain their condition against occlusions, angiopathy, etc.

The basic concept of the stent is well known.

Over time various stents manufactured from biocompatible materials have been proposed. Usually these are of a tubular armature that can be a self-expanding elastic type, a compressed spring type, a mechanical or thermal dilation type or a memory type or similar.

The structure of a stent is generally reticular, with links obtained with a shear, or with a laser cutter, from a continuous sheet, or from a tube, or formed from wire coil, or bent in a sinusoidal, helicoidal or zigzag form, variously joined, crossed or welded. The links can be of various forms, but normally they are all equal and are uniformly repeated throughout the structure. Moreover, the links are joined one to the next, to all or in groups.

Some examples of known stents are described and laid claim to in the following Patents:
US-5 041 126; US-5 161 547; US-5 217 483; DE-4 303 181; DE-4 407 079; DE-4 240 177; EP-0 421 729; US 5 135 536.

When they are manufactured starting from a wire they are normally mono-component, in the sense that they are produced from a single material such as platinum, gold, etc., or of an alloy of metals.

In any case, the structures of the known stents are not without their problems and inconveniences, among which can be mentioned the irregularity or the limitation of the expansion, because of the fact that the links are all, or the major part are, fixed one to the other; the impossibility of varying the elasticity or the degree of expansion between one part and another part of the structure, in that it acts the same for all the structure.

It is the aim of the present invention to furnish a new, original stent structure which is able to reduce, if not to fully eliminate, the problems and the inconveniences associated with presently known stents.

To this purpose a structure has been arrived at for an intravascular radially expandable stent conforming to that in Claim 1.

Greater detail of the invention, such as its advantages, will be more evident from the following description, made with reference to the attached drawings, in which:
Fig. 1 shows an enlarged section of the initial wire used for the manufacture of the stent, the subject of this invention;
Fig. 2 shows the initial wire when it is in the corrugated form before being wound into a tubular form;
Fig. 3 shows the stent when it is configured for use;
Fig. 4 shows, enlarged, some undulations or corrugations to highlight the form; and
Fig. 5 shows a cross-section of the wire forming the stent, on the line V-V in Fig. 4,

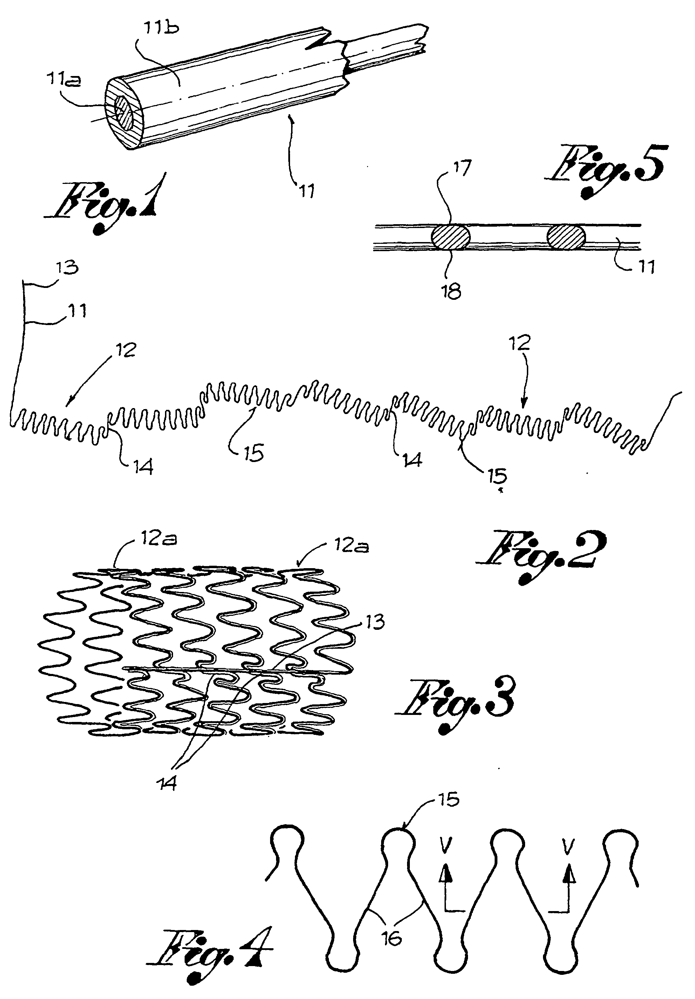

The stent proposed here starts with a wire 11, which is corrugated to form a sequence of corrugated sections 12 -Fig. 2- leaving a tail 13 at least at one end, and then winding the wire corrugated in this way around a virtual axis to form a tubular structure.

The initial wire 11, can be a mono-component or, preferably, bi-component. In this latter case it consists of a filiform core 11 a in one material and an external layer or covering in a second material, enclosing the filiform core 11 a and closely mating with it.

The filiform core can be in a material such as tantalum, for example; the external covering can be in another biomedical material, such as MP35N, or other alloy, which will anyway be weldable.

The corrugated elements 12 are connected consecutively in sequence by rectilineal portions 14 and include a number of single wave forms 15 substantially equal. These wave forms 15 have curved crests and troughs, semicircular or greater than semicircular connected by rectilineal divergent wire sections 16 as shown in Fig 4. This imparts to the stent a greater dimensional stability than with the usual sinusoidal form of initial wire.

Then, preferably, the wire 11 is pressed or flattened on two opposite sides 17, 18 to impart an oval form to its cross-section -Fig. 5-.

This operation is preferably performed after the corrugation so that the flattened sides 17, 18 of the wire are parallel planes to the plane containing the corrugations and become the internal and external surfaces of the tubular structure into which the wire is then formed. This configuration permits, sections being equal, an increase in the external contact area with the vessel in which it is implanted and an increase in the internal bore of the stent, with a reduction in thrombogenic tendency compared to a stent produced from a tube.

This tubular structure -Fig 3- is obtained by winding the corrugated wire helicoidally around a virtual axis. This is done in such a way that the sequence of corrugated segments 12 form the consecutive coils 12a of the resulting tubular structure, connected one to the other by the intermediate rectilineal portions 14.

The corrugations of the consecutive coils are not fixed to each other; they can be simply close to one another but without being joined, maintaining in this way the maximum degree of freedom.

To maintain the tubular form of the stent, some or all of the coils are attached by welding to a length of the initial wire tail 13 which is bent to extend it substantially along the generatrix of the structure, externally or internally -see Fig. 3-. Preferably, the tail of the wire is welded to the rectilineal portions 14 that connect the consecutive coils. The coils of the stent can also be fixed to an additional length of wire laid longitudinally.

The resulting structure of the stent gives the benefit of maximum contraction during the implantation phase and then of dilation when implanted, taking full advantage of the elasticity of the singular corrugations of the wire and of the whole structure.

In addition, the bi-component initial wire offers the possibility, also through a partial annealing, together with the ovalization of the section, of modifying the mechanical strength, flexibility and elasticity properties of the stent. It is also possible to impart different elasticity properties to different zones of the stent, depending on the requirements and to better employ the stent with whatever means of introduction and expansion being used, for example, through an inflatable balloon or to a form memory.

When the initial wire is bi-component, the external covering can be welded where necessary without damaging the internal core and so without altering the specific properties of the structure even in the presence of welding shock.

In conclusion, the structure and the behaviour of the stent is improved as is the adaptability of the stent in use.

## Claims

1. A radially contractible and expandable stent structure constructed of an initial wire (11) corrugated to form a sequence of corrugated segments (12), with at least one terminal tail (13), said corrugated segments (12) being wound helicoidally around a virtual axis to form a tubular structure in which said segments form consecutive coils, at least one of said terminal tails (13) being bent to extend and to be welded along the length of a generatrix of the tubular structure so joining the coils one to the other for the stability of the structure, **characterised by** the fact that said segments are connected consecutively by intermediate rectilineal portions (14), said coils are connected to each other by said intermediate rectilineal portions, said corrugated segments (12) are equal and each one consists of a number of single corrugations substantially equal and having crests and troughs connected by lengths of diverging rectilineal wire (16), the crests and troughs being semicircular or greater than semicircular.

2. A structure of stent in accordance with claim 1, in which the tie tail (13) of the resulting tubular structure extends parallely and is fixed to the intermediate rectilineal connecting portions (14) of the consecutive coils, the tail being able to pass externally or internally along the structure.

3. A stent structure in accordance with claim 2, in which at least some of the coils of the tubular structure are fixed to lengths of additional wire placed longitudinally and spaced around the said tubular structure.

4. A stent structure in accordance with the previous claims, in which the initial wire (11) is pressed or flattened on two opposite sides for the ovalization of its section, the flattening being effected after the corrugating of the wire and the flattened faces result in being parallel planes to the corrugation plane of the wire.

5. A stent structure in accordance with any of the previous claims, in which the initial wire is mono-component.

6. A stent structure in accordance with any of the claims from 1 to 3, in which the initial wire is composed of an internal core (11a) in one material and an external layer or covering in another material.

7. A stent structure in accordance with claim 1, in which said lengths of rectilineal wire (16) diverge one from the other in the direction from said crests to said troughs.

## Patentansprüche

1. Eine radial zusammenziehbare und ausdehnbare Stentstruktur, die aus einem Anfangsdraht (11) aufgebaut ist, der gewellt ist, um eine Abfolge von gewellten Segmenten (12) zu bilden, mit zumindest einem endseitigen Schlussstück (13), wobei die gewellten Segmente (12) schraubenförmig um eine virtuelle Achse gewickelt sind, um eine rohrförmige Struktur zu bilden, bei der die Segmente aufeinander folgende Windungen bilden, wobei zumindest eines der endseitigen Schlussstücke (13) gebogen ist, um sich entlang der Länge einer Generatrix der rohrförmigen Struktur zu erstrecken und entlang derselben geschweißt zu sein und so die Windungen zur Stabilität der Struktur aneinander zu fügen, **dadurch gekennzeichnet, dass** die Segmente durch geradlinige Zwischenstücke (14) aufeinander folgend verbunden sind, die Windungen durch die geradlinigen Zwischenstücke miteinander verbunden sind, die gewellten Segmente (12) gleich sind und jedes aus einer Anzahl von einzelnen Wellungen besteht, die im wesentlichen gleich sind und Scheitel und Täler aufweisen, die durch Längen eines auseinander gehenden geradlinigen Drahtes (16) verbunden sind, wobei die Scheitel und Täler halbkreisförmig oder größer als halbkreisförmig sind.

2. Eine Stentstruktur nach Anspruch 1, bei der sich das Bindeschlussstück (13) der resultierenden rohrförmigen Struktur parallel erstreckt und an den geradlinigen Zwischenverbindungsstücken (14) der aufeinander folgenden Windungen befestigt ist, wobei das Schlussstück in der Lage ist, extern oder intern entlang der Struktur zu verlaufen.

3. Eine Stentstruktur nach Anspruch 2, bei der zumindest einige der Windungen der rohrförmigen Struktur an Längen von zusätzlichem Draht befestigt sind, der in Längsrichtung platziert und um die rohrförmige Struktur herum angeordnet ist.

4. Eine Stentstruktur nach den vorhergehenden Ansprüchen, bei der der Anfangsdraht (11) zur Ovalisierung seines Querschnittes an zwei gegenüberliegenden Seiten zusammengedrückt oder abgeflacht ist, wobei das Abflachen nach dem Wellen des Drahtes erfolgt und die abgeflachten Flächen somit parallele Ebenen zu der Wellungsebene des Drahtes sind.

5. Eine Stentstruktur nach einem der vorhergehenden Ansprüche, bei der der Anfangsdraht monokomponent ist.

6. Eine Stentstruktur nach einem der Ansprüche 1 bis 3, bei der der Anfangsdraht aus einem inneren Kern (11a) aus einem Material und einer äußeren Schicht oder Abdeckung aus einem anderen Material zusammengesetzt ist.

7. Eine Stentstruktur nach Anspruch 1, bei der die Längen des geradlinigen Drahtes (16) in die Richtung von den Scheiteln zu den Tälern auseinander gehen.

## Revendications

1. Structure d'un stent expansible et pouvant se contracter radialement se composant d'un fil initial (11) ondulé pour former une séquence de segments ondulés (12), avec au moins une queue terminale (13), lesdits segments ondulés (12) étant enroulés de manière hélicoïdale autour d'un axe virtuel pour former une structure tubulaire dans laquelle lesdits segments forment des bobines consécutives, au moins une desdites queues terminales (13) étant pliée pour s'étendre et être soudée le long de la longueur d'une génératrice de la structure tubulaire joignant ainsi les bobines les unes aux autres pour la stabilité de la structure, **caractérisée par le fait que** lesdits segments sont reliés de manière consécutive par des parties rectilignes intermédiaires (14), lesdites bobines sont reliées les unes aux autres par lesdites parties rectilignes intermédiaires, lesdits segments ondulés (12) sont égaux et chacun se compose d'un certain nombre de simples ondulations sensiblement égales et ayant des crêtes et des creux semi-circulaires ou plus que semi-circulaires.

2. Structure d'un stent selon la revendication 1, dans laquelle la queue d'attache (13) de la structure tubulaire résultante s'étend de manière parallèle et est fixée aux parties de raccordement rectilignes intermédiaires (14) des bobines consécutives, la queue pouvant passer de manière externe ou interne le long de la structure.

3. Structure d'un stent selon la revendication 2, dans laquelle au moins certaines des bobines de la structure tubulaire sont fixées aux longueurs du fil supplémentaire placé longitudinalement et espacé autour de ladite structure tubulaire.

4. Structure d'un stent selon les revendications précédentes, dans laquelle le fil initial (11) est pressé ou aplati sur deux côtés opposés pour l'ovalisation de sa section, l'aplatissement étant effectué après l'ondulation du fil et les faces aplaties résultent sous forme de plans parallèles par rapport au plan d'ondulation du fil.

5. Structure d'un stent selon l'une quelconque des revendications précédentes, dans laquelle le fil initial est mono-composant.

6. Structure d'un stent selon l'une quelconque des revendications 1 à 3, dans laquelle le fil initial est composé d'un noyau intérieur (11a) dans un matériau et d'une couche extérieure ou d'un revêtement dans un autre matériau.

7. Structure d'un stent selon la revendication 1, dans laquelle lesdites longueurs de fil rectiligne (16) divergent les unes des autres dans la direction allant desdites crêtes auxdits creux.
